(19) Europäisches Patentamt
European Patent Office
Office européen des brevets

(11) Publication number: **0 064 165**
**B1**

(12) **EUROPEAN PATENT SPECIFICATION**

(45) Date of publication of patent specification: **10.06.87**

(21) Application number: **82102950.1**

(22) Date of filing: **06.04.82**

(51) Int. Cl.$^4$: **C 07 D 311/26**, C 07 D 311/36, C 07 D 311/30, A 61 K 31/35

(54) **Antihypertensive agents.**

(30) Priority: **01.05.81 US 259403**

(43) Date of publication of application: **10.11.82 Bulletin 82/45**

(45) Publication of the grant of the patent: **10.06.87 Bulletin 87/24**

(84) Designated Contracting States: **AT BE CH DE FR GB IT LI LU NL SE**

(56) References cited:
**CH-A- 378 339**
**FR-A-1 320 300**
**US-A-3 340 266**

**Act. Pharm. Sin. 15, 253 (1980)**

(73) Proprietor: **PENNWALT CORPORATION**
**Pennwalt Building Three Parkway**
**Philadelphia Pennsylvania 19102 (US)**

(72) Inventor: **Wu, Edwin Shen-Chou**
**147 Ashley Drive**
**Rochester New York 14620 (US)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 064 165 B1

**Description**

This invention relates to chromone derivatives substituted by 3-amino-2-hydroxypropoxy side chains, medicinal preparations containing the same, and the utilization of such preparations as antihypertensive agents in animals.

Compounds related to propranolol, a potent beta-blocker, constitute a large class of compounds which have profound effects on the cardiovascular system and have found utility as antihypertensive, antidysrhythmic and antianginal drugs. However, the beta-blocking properties of these compounds are often undesirable, especially in patients with coronary insufficiencies and bronchial diseases. A compound devoid of beta-blocking effect but retaining antihypertensive effect on the blood pressure of warm-blooded animals has long been sought.

Several patents disclose various compounds which either have beta-blocking properties or do not have antihypertensive effects, US—A—3,891,651 discloses compounds which are amides and, it is thought, the nitrogen of the amide which is contained in the isoquinoline fragment is likely to be responsible for any activity in that compound. US—A—3,816,470 discloses various salts of secondary amines with chromone-2-carboxylic acids. US—A—3,812,156 discloses a method of preparing ethyl flavone-7-oxyacetate, US—A—3,352,754 discloses simple 7-hydroxy or 7-alkoxy isoflavones which are not amines and which are used for various inflammatory disorders. US—A—3,219,531 discloses 5,7-dioxyacetic acid flavone compounds, but no amine functions are present. US—A—3,046,275 disloses 7-dialkylaminoalkoxy derivatives but does not contain any of the hydroxyl groups of the side chain which is central for activity. Various monodialkylaminoethyl ethers of quercitin are disclosed in US—A—2,861,992, but do not contain 3-amino-2-hydroxypropoxy side chains. Also not containing that side chain are the compounds disclosed in US—A—2,897,211.

CH—A—378,339 discloses certain 7-dialkylaminoalkoxyflavones possessing antispasmodic, muscle relaxant and coronary vasodilator activities.

FR—A—1,320,300 discloses 7-(3-dialkylaminopropoxy)-isoflavones and 7-(carbamyl or alkoxycarbonyl, or carboxy)alkoxyisoflavones.

US—A—3,340,266 discloses among others dihydrochromones useful for the treatment of dysrhythmias and angina pectoris. Several flavones have been published in Acta Pharmaceutica Sinica, 15, *253* (1980). However, these compounds are excluded from the subject-matter of the present invention.

It has now been found that a marked reduction in the blood pressure of warm-blooded animals can be achieved, without beta-blocking effects in the beta-adrenergic nervous system, by administering to the animal, in an amount effective to reduce hypertension, a chromone of the formula:

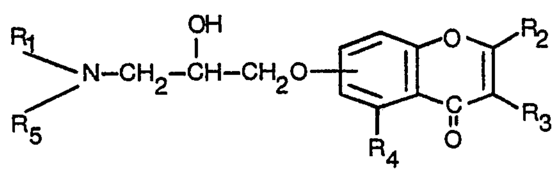

or a pharmaceutically acceptable salt thereof wherein the $R_1R_5N—CH_2—CH(OH)CH_2O$ group is in the 6 or 7 position of the chromone nucleus; $R_1$ is H, primary or secondary alkyl of 1 to 4 carbon atoms, or cycloalkyl of 3 to 6 carbon atoms; $R_2$ is H, lower alkyl comprising 1 to 4 carbon atoms, $CF_3$, phenyl, o-chlorophenyl or p-chlorophenyl; $R_3$ is H or phenyl or benzyl; $R_4$ is hydrogen or hydroxyl; $R_5$ is hydrogen or methyl; provided, however, that at least one of $R_2$ or $R_3$ is phenyl or substituted phenyl, and that $R_5$ is methyl only if $R_1$ is isopropyl, $R_2$ is methyl, $R_3$ is phenyl and $R_4$ is hydrogen or $R_1$ is methyl, $R_2$ is phenyl, $R_3$ is phenyl and $R_4$ is hydrogen.

These compounds are able to achieve long-term activity (e.g., 24 hours) at relatively low dosages.

The compounds are usually mixed with a pharmaceutical carrier so that the composition for commercial use contains 0.5 to 20% by weight of the compound.

The compositions are normally adapted for peroral or parenteral use, but may be used in other forms such as suppositories. The peroral compositions are preferably in the form of tablets, capsules or suspensions, while the parenteral composition is preferably an injectable solution or suspension.

Examples of suitable inert pharmaceutical carriers are celluloses (particularly microcrystalline celluloses), sugar syrups, potato starch, talcum, polyethylene glycols and lactose.

Examples of suitable acids for forming the acid addition salts are maleic acid, hydrochloric acid, sulfuric acid, phosphoric acid, methanesulfonic acid, tartaric acid, citric acid, and the cation exchange resins such as the carboxylic acid, phosphonic acid and sulfonic acid resins.

For sustained release, a coated complex of the compound absorbed onto an ion exchange resin may be employed in accordance with the teaching of GB—A—1,544,761.

The usual peroral dosage of the compound is 0.1 to 150 mg. per day (preferably 0.1 to 50 mg.) while the parenteral dosage is normally 0.1 to 40 mg. per day (preferably 0.1 to 10 mg.).

The capsules, tablets, syrups and suspensions of the compounds are prepared by conventional procedures.

It should be noted that the compounds of this invention are antihypertensive agents, not hypotensive agents, i.e., they reduce the blood pressure to normal but not below normal.

The compounds of formula (1) above can be prepared by reacting epichlorohydrin or epibromohydrin with a compound of the formula

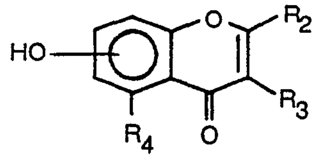

wherein $R_2$, $R_3$ and $R_4$ are the same as defined above and the hydroxyl group is substitued on the 6 or 7 position, in the presence of a solvent and a base to give an epoxide of the above formula where the hydroxyl group is replaced by

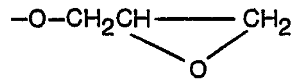

and reacting this epoxide with an amine of the formula $R_1R_5NH$, where $R_1$ and $R_5$ are as above defined, in an alcoholic solvent at elevated temperature and thereafter optionally converting the obtained compound into a pharmaceutically acceptable salt thereof.

P. Da Re et al, *J. Med. Chem.,* Vol. 15, 868—869 (1972), describe the testing of chromones as in formula (1), but where $R_3$ is methyl, for beta-adrenergic blocking activity. Da Re et al. found that all the compounds were devoid of beta-blocking activity, suggesting that chromones would not be expected to have antihypertensive properties. Additionally, ethanolamine analogues of the Da Re et al. materials are disclosed in Vol 15. pages 198—199 of the *J. Med. Chem.* (1972). These analogues are beta-blockers, typical of the pronethalol type which owe their activity to the 2-isopropylaminoethanol side chain. Typical 3-amino-2-hydroxypropoxy side chain furochromone compounds are disclosed in papers presented in *Drugs of the Future,* Vol. III, No. 8 (1978), pages 596—571; *Drugs of the Future,* Vol. III, No. 11, (1978), pages 816—818; and *Therapie,* (1977), Vol. 32, pages 111—120. None of these references, of course, even suggest that antihypertensive activity may be possible with or without beta-blocking. None of these references discloses the compounds of this invention.

The following general procedures are used in the examples to follow:

*A. For Epoxides* — Epichlorohydrin or epibromohydrin (greater than 2 equivalents) is added in the presence or absence of nitrogen to a stirred solution or suspension of the hydroxy chromone derivative in solvents, such as acetone, aqueous ethanol, 50% aqueous dimethylsulfoxide (DMSO) or water, containing a suitable base, such as potassium carbonate or sodium hydroxide, with or without sodium iodide. The reaction is allowed to proceed either at room temperature or elevated temperature and monitored by thin layer chromatography (tlc). Product formed as solid is then collected and washed with water. The mother liquor is diluted with water and extracted with chloroform ($CHCl_3$) to give more product. Where the product is soluble in solvents, the reaction mixture is filtered off and the solids washed with the solvent. The filtrate is evaporated to give a solid which is washed with water to get rid of remaining traces of the base. Yield is in the 60 to 80% range. This material is usually used directly for further reaction without purification.

*B. For Epoxide Ring-Opening With Amine and the Amine Salt Formation* — A white suspension of the chromone epoxide, amine, such as low (large excess) and high (10% excess) boiling point amines, and alcoholic solvent, methanol (A.R.) ethanol (abs.) or isopropanol, is heated at elevated temperatures until the starting material is gone (as followed by tlc.) Since the reaction product is usually soluble in the alcohol, the precipitate is filtered off and the filtrate is evaporated to give a viscous liquid which, upon addition of anhydrous ether or alcohol, crystallizes out. When the product is insoluble in alcohol at the end of the reaction, it is collected. Purification of the amine derivative is performed via acid-base work-up, column chromatography or recrystallization. The amine obtained is either suspended or dissolved in an alcohol, such as MeOH or abs. ethanol (EtOH) or isopropanol, and then acidified with alcoholic solution saturated with hydrogen chloride or another suitable acid. The salt formed is precipitated out by addition of anhydrous ether. The salt is then recrystallized from suitable solvents.

Example 1

Preparation of 7-(2-hydroxy-3-isopropylaminopropoxy)-2,3-diphenylchromone hydrochloride:

*7-(2,3-Epoxypropoxy)-2,3-diphenylchromone* — To a solution of 50% aqueous DMSO (280 ml) containing sodium hydroxide (5.60 g, 140 mmol) was added 7-hydroxy-2,3-diphenylchromone (44.01 g, 140 mmol) and the resultant mixture was stirred until most of the solid went into the solution. Epichlorohydrin was then introduced into the suspension. The solution was stirred at room temperature (RT) for 2.5 days and solid formed during the reaction was collected from time to time (about twice a day). The white solid (total = 47.58 g) was recrystallized from isopropyl alcohol several times to afford 28.15 g of relatively pure white prisms, m.p. 143—145°; yield 54%. The mother liquor was concentrated to produce more crystals.

*7-(2-Hydroxy-3-isopropylaminopropoxy)-2,3-diphenylchromone hydrochloride* — A white suspension of relatively pure 7-(2,3-epoxypropoxy)-2,3-diphenylchromone (27.78 g, 75 mmol), isopropylamine (18.8 ml), and methanol (A.R., 150 ml) was heated under nitrogen at 55°C until complete disappearance of the starting epoxide. The white precipitate (1.09 g) in the cooled reaction mixture was filtered off, and the filtrate was evaporated to a golden syrup. Anhydrous ether was added, and white crystals (28.07 g) were obtained. It was column-chromatographed over silica gel. This purification yielded 24.05 g of white crystals, m.p. 152—154° (75%).

The amine thus obtained was dissolved in 100 ml of MeOH A.R. and treated with saturated solution of hydrogen chloride in ethanol until the solution was pH = 1. The hydrochloride salt was precipitated out by addition of anhydrous ether. A white solid was collected and recrystallized from MeOH-ether (also decolorized with a small amount of activated charcoal) to produce 21.6 g (83% yield) of pure white crystals, m.p. 164—166°.

| | | | | |
|---|---|---|---|---|
| Anal. Calc'd. for $C_{27}H_{28}ClNO_4.H_2O$: | C, 67.00; | H, 6.25; | Cl, 7.32; | N, 2.89. |
| Found: | C, 67.10; | H, 5.95; | Cl, 7.19; | N, 2.65. |

Example 2

Preparation of 7-(2-hydroxy-3-isopropylaminopropoxy) flavone hydrochloride

*7-(2,3-Epoxypropoxy)flavone* — Epichlorohydrin (231 g, 195.6 ml, 2.5 mol) was slowly added to a stirred yellow solution of 7-hydroxyflavone (119.0 g, 0.5 mol) in 50% aq EtOH (500 ml) containing NaOH (20.0 g, 0.5 mol) and left to stand at RT. It was left overnight and the white solid, 89.9 g, m.p. 252—4° (with some solid melting around 120°), was collected and washed with water. The original filtrate of the reaction mixture before water washing was stirred at RT overnight, yielding 73.6 g white solid after filtration and washing with water (at that time, tlc, 2% methanol in chloroform, indicated that only a small amount of the starting flavone was left).

The first crop of the solid was suspended in chloroform (ca 250 ml), stirred for 30 min., and filtered. Evaporation of the chloroform solution afforded the desired epoxide as white crystals (21.12 g) m.p. 121—124°. Similarly, the second crop of the white solid gave 49.97 g of the epoxide m.p. 119—121°.

The water washings from the first and second crops were combined and extracted with chloroform. The chloroform extracts were combined and evaporated to yield a white solid, which was washed with water to give 3.11 g, m.p. 114—115°.

Water was added to the mother liquor from the second crop, and solid was collected. The solid was added to the filtrate, and it was extracted with chloroform twice. The extracts were evaporated to leave a reddish-gold oil which crystallized upon being seeded. The sticky solid was collected and washed with a small amount of anhydrous ether to produce 12.53 g of white crystals, m.p. 120—125°. Total yield, 86.7 g, is 59%.

It can also be prepared in 68% yield by refluxing under nitrogen 7-hydroxyflavone, epichlorohydrin or epibromohydrin (2 eq), anhydrous potassium carbonate (3 eq), and NaI (1.5 eq) in acetone for about 2 days.

*7-(2-Hydroxy-3-isopropylaminopropoxy)flavone hydrochloride* — A suspension of 7-(2,3-epoxypropoxy)flavone 19.0 g, 0.065 mol) and isopropylamine (23.05 g, 33.2 ml, 0.39 mol) in methanol (AR, 250 ml) was stirred under nitrogen at RT. After 1.5 days' stirring, the reaction was not completed yet, as evidenced by tlc. It was then heated at 60° for 12 hrs. (to drive the reaction to completion). The solvent and excess of isopropylamine were removed under reduced pressure, thus leaving yellow crystals which, upon washing with anhydrous ether, gave a white solid (14.64 g), m.p. 140—142°.

The ether washing was evaporated and a yellow sticky solid obtained. It was washed with a small amount of ether, but it remained sticky. Therefore, it was dissolved in a small amount of MeOH (AR) and treated with HCl-saturated EtOH until acidic. The solvents were removed, and the residue was dissolved in water (10 ml) and aq 10% HCl, extracted with chloroform (30 ml × 2). The aqueous layer was basified with conc. ammonia and extracted with chloroform (30 ml × 4). Chloroform extracts were washed with water and saturated NaCl, and dried ($MgSO_4$), thus affording 1.0 g of light brown crystals, m.p. 140—142°. Combined yield, 15.64 g, was 68%.

The free base (14.64 g) in MeOH (AR, 150 ml; a suspension) was treated with HCl-saturated ethanol (abs 2B) dropwise. It became a solution when the mixture was acidic, and then pale yellow crystals precipitated out. MeOH was added to make it to 400 ml and heated to bring it to a solution. It was concentrated to 250 ml, and anhydrous ether (800 ml) was added and cooled to give pale yellow prisms (15.92 g, 98.6% yield), m.p. 231—233°. The yellow color was gone upon drying at 80° under vacuum.

| | | | | | |
|---|---|---|---|---|---|
| Anal. Calc'd. for $C_{21}H_{24}ClNO_4$: | C, 64.69; | H, 6.20; | Cl, 9.09; | N, 3.59; | O, 16.41. |
| Found: | C, 64.68; | H, 6.20; | Cl, 9.02; | N, 3.56; | O, 16.52. |

Example 3

Preparation of 7-(2-hydroxy-3-isopropylaminopropoxy)-3-phenylchromone hydrochloride:

*7-(2,3-Epoxypropoxy)-3-phenylchromone* — 7-Hydroxy-3-phenylchromone and epichlorohydrin were reacted according to general procedure A, and the product obtained was white prisms, m.p. 151—152° (83% crude yield) from isopropyl alcohol.

*7-(2-Hydroxy-3-isopropylaminopropoxy)-3-phenylchromone hydrochloride* — 7-(2,3-Epoxypropoxy)-3-phenylchromone and isopropylamine were reacted according to Method B to give the product as the hydrochloride in 62% yield, mp. 148—150° (EtAC-EtOH-Ether). Its corresponding free base, m.p. 128—130°.

| | | | | |
|---|---|---|---|---|
| Anal. Calc'd. for $C_{21}H_{24}ClNO_4$: | C,64.69; | Cl, 9.09; | H, 6.20; | N, 3.59. |
| Found: | C, 64.63; | H, 6.30; | Cl, 9.65; | N, 3.56. |

Example 4

Preparation of 7-(3-*n*-propylamino-2-hydroxypropoxy) flavone hydrochloride:

Using Method B, the reaction of 7-(2,3-epoxypropoxy)-flavone and *n*-propylamine afforded the hydrochloride in 48% yield, m.p. 234—235° (methanol). Its corresponding free base had a m.p. 140—145°.

| | | | | | |
|---|---|---|---|---|---|
| Anal. Calc'd. for $C_{21}H_{24}ClNO_4$: | C, 64.69; | H, 6.20; | Cl, 9.09; | N, 3.59; | O, 16.41. |
| Found: | C, 64.60; | H, 6.37; | Cl, 8.44; | N, 3.48; | O, 16.57. |

Example 5

Preparation of 2′-chloro-7-(2-hydroxy-3-isopropylaminopropoxy) flavone

*2′-Chloro-7-(2,3-epoxypropoxy)-flavone* — The epoxypropoxy ether was prepared in quantitative yield (crude) from 2′-chloro-7-hydroxyflavone according to Method A.

*2′-Chloro-7-(2-hydroxy-3-isopropylaminopropoxy) flavone hydrochloride* — The amine was prepared from the epoxide and isopropylamine according to Method B. The yield of recrystallized product (from toluene) was 56%, melting at 114—116°.

| | | | | | |
|---|---|---|---|---|---|
| Anal. Calc'd. for $C_{21}H_{22}ClNO_4$: | C, 65.03; | H, 5.71; | Cl, 9.14; | N, 3.61; | O, 16.41. |
| Found: | C, 65.22; | H, 6.00; | Cl, 9,19; | N, 3.56; | O, 15.55. |

Example 6

Preparation of 6-(2-Hydroxy-3-isopropylaminopropoxy) flavone hydrochloride:

*6-(2,3-Epoxypropoxy)flavone* — This epoxypropyl ether was obtained in 65% yield from 6-hydroxyflavone according to Method A.

*6-(2-Hydroxy-3-isopropylaminopropoxy) flavone hydrochloride* — The reaction of the above epoxide with isopropylamine according to Method B yielded (after recrystallization from *n*-butanol) 49% product, melting at 209—211°.

| | | | | | |
|---|---|---|---|---|---|
| Anal. Calc'd. for $C_{21}H_{24}ClNO_4$: | C, 64.69; | H, 6.20; | Cl, 9.09; | N, 3.59; | O, 16.41. |
| Found: | C, 64.59; | H, 6.61; | Cl, 9.64; | N, 3.40; | O, 16.02. |

Example 7

Preparation of 4′-chloro-7-(2-hydroxy-3-isopropylaminopropoxy) flavone hydrochloride

*4′-Chloro-7-(2,3-epoxypropoxy)flavone* — The epoxypropyl ether was prepared in quantitative yield (crude) from 4′-chloro-7-hydroxyflavone according to Method A.

*4′-Chloro-7-(2-hydroxy-3-isopropylaminopropoxy) flavone hydrochloride* — The amine hydrochloride was prepared from the above epoxide and isopropylamine using Method B. The yield of recrystallized product from dimethylformamide (DMF) was 63%, m.p. 258—259°.

| | | | | | |
|---|---|---|---|---|---|
| Anal. Calc'd. for $C_{21}H_{23}Cl_2NO_4$: | C, 59.44; | H, 5.46; | Cl, 16.71; | N, 3.30; | O, 15.08. |
| Found: | C, 59.35; | H, 5.60; | Cl, 16.96; | N, 3.44; | O, 15.21. |

Example 8

Preparation of 7-(3-*n*-propylamino-2-hydroxypropoxy)-2,3-diphenylchromone hydrochloride

The free base was prepared according to Method B from *n*-propylamine and 7-(2,3-epoxypropoxy)-2,3-diphenylchromone in 65% yield as white prisms, m.p. 140—142°. (*i*PrOH).

The hydrochloride salt was obtained in 95% yield as white prisms, m.p. 134—136° (*i*PrOH).

Example 9

Preparation of 7-(3-cyclopropylamino-2-hydroxypropoxy)flavone hydrochloride:

Using Method B, the amine was prepared from cyclopropylamine and 7-(2,3-epoxypropoxy) flavone and purified by column chromatography, yield 26%. Treatment of the free base with HCl/EtOH solution gave the hydrochloride salt in 95% yield, m.p. 214° (MeOH-ether).

| | | | | | |
|---|---|---|---|---|---|
| Anal. Calc'd. for $C_{21}H_{22}ClNO_4$: | C, 65.03; | H, 5.71; | Cl, 9.14; | N, 3.61; | O 16.49. |
| Found: | C, 64.82; | H, 5.81; | Cl, 9.27; | N, 3.65; | O, 16.78 |

### Example 10

Preparation of 7-(3-Methylamino-2-hydroxypropoxy) flavone hydrochloride:

The free amine was prepared according to Method B from 7-(2,3-epoxypropoxy) flavone and 40% aqueous methylamine in 30% yield after column purification, m.p. 133—4° (MeOH-$CH_2Cl_2$). The hydrochloride salt, m.p. 251—52° (MeOH); yield 68% (purified).

| | | | | | |
|---|---|---|---|---|---|
| Anal. Calc'd. for $C_{19}H_{20}ClNO_4.\frac{1}{2}H_2O$: | C, 61.54; | H, 5.71; | Cl, 9.56; | N, 3.78; | O, 19.42. |
| Found: | C, 61.47; | H, 5.82; | Cl, 9.88; | N, 3.63; | O, 19.92. |

### Example 11

Preparation of 7-(3-Ethylamino-2-hydroxypropoxy) flavone hydrochloride:

According to Method B, the title compound was synthesized from the reaction of 7-(2,3-epoxypropoxy) flavone and 70% aqueous ethylamine followed by treatment with HCl/EtOH, as white prisms, m.p. 244—46° (MeOH) in 36% yield. The free base was obtained after column purification as white crystals, m.p. 164—65° (MeOH/ether), yield 39%.

| | | | | | |
|---|---|---|---|---|---|
| Anal. Calc'd. for $C_{20}H_{22}ClNO_4$: | C, 63.91; | H, 5.89; | Cl, 9.43; | N, 3.72; | O, 17.02. |
| Found: | C, 64.07; | H, 6.03; | Cl, 9.65; | N, 3.76; | O, 17.14. |

### Example 12

Preparation of 7-(3-*n*-butylamino-2-hydroxypropoxy) flavone hydrochloride

Using Method B, the reaction of 7-(2,3-epoxypropoxy) flavone and *n*-butylamine afforded the hydrochloride in 35% yield (purified) as white crystals, m.p. 224—25° (MeOH). The free base was purified by high pressure liquid chromatography as an off-white powder, m.p. 143—44° (53% yield).

| | | | | | |
|---|---|---|---|---|---|
| Anal. Calc'd. for $C_{22}H_{26}ClNO_4$: | C, 65.42; | H, 6.48; | Cl, 8.77; | N, 3.46; | O, 15.84. |
| Found: | C, 65.01; | H, 6.83; | Cl, 9.08; | N, 3.33; | O, 15.41. |

### Example 13

Preparation of 7-(3-cyclohexylamino-2-hydroxypropoxy) flavone hydrochloride:

The title compound was prepared according to Method B in 63% yield as a white powder, m.p. 242—43° (MeOH). Its free base was purified by high pressure liquid chromatography and obtained as white powder, m.p. 174—76° (63% yield).

| | | | | |
|---|---|---|---|---|
| Anal. Calc'd. for $C_{24}H_{28}ClNO_4.\frac{1}{2}H_2O$: | C, 65.67; | H, 6.66; | N, 3.19; | O, 16.40. |
| Found: | C, 65.34; | H, 6.87; | N, 3.16; | O, 16.86. |

### Example 14

Preparation of 7-(3-cyclopropylamino-2-hydroxypropoxy)-2,3-diphenylchromone:

The amine was prepared according to Method B from cyclopropylamine and 7-(2,3-epoxypropoxy)-2,3-diphenylchromone and purified by high pressure liquid chromatography to give white crystals, m.p. 135—137° (MeOH-ether), in 25% yield.

### Example 15

Preparation of 7-(3-amino-2-hydroxypropoxy) flavone hydrochloride:

7-(2,3-Epoxypropoxy)flavone (13.3 g, 0.045 mol) in a pressure bottle was treated with a methanolic solution (90 ml) containing 1.87 g of ammonia (0.11 mol). The bottle was sealed and the suspension was heated at 50° for 12 hrs. A tlc (15% MeOH/$CHCl_3$) indicated that the starting material was gone, and the reaction mixture was cooled and filtered. The solid (5.32 g) thus obtained was shown by tlc to be a byproduct, m.p. 209—12°. The filtrate, upon evaporation, afforded 7.0 g of a yellow solid, m.p. 121—27°, which was purified by high pressure liquid chromatography to give 1.85 g of yellow powder, m.p. 154—56°. (13% yield). The hydrochloride salt was prepared and recrystallized from MeOH, m.p. 191—2°.

### Example 16

Preparation of 7-(3-propylamino-2-hydroxypropoxy)-3-benzylflavone hydrochloride:

*3-Benzyl-7-(2,3-epoxypropoxy)flavone* — Using Method A, 3-benzyl-7-hydroxyflavone was converted to its corresponding epoxide, m.p. 133—138°.

*7-(2-hydroxy-3-propylaminopropoxy)-3-benzylflavone hydrochloride* — The amine salt was prepared from 3-benzyl-7-(2,3-epoxypropoxy)flavone as described in Method B as white prisms, m.p. 196—8° (*i*PrOH); yield, 50% (from the epoxide).

| | | | | |
|---|---|---|---|---|
| Anal. Calc'd. for $C_{28}H_{30}ClNO_4$: | C, 70.07; | H, 6.30; | Cl, 7.39; | N, 2.92. |
| Found: | C, 69.78; | H, 6.32; | Cl, 7.49; | N, 2.74. |

Example 17

Preparation of 7-(2-hydroxy-3-isopropylaminopropoxy)chrysin hydrochloride:

*5-Hydroxy-7-(2,3-epoxypropoxy)flavone* — Using Method A, the epoxide was prepared as a light yellow solid from chrysin hydrochloride in 26% yield (purified); m.p. 169—170°.

| | | | |
|---|---|---|---|
| Anal. Calc'd. for $C_{18}H_{13}O_5$: | C, 69.89; | HG, 4.23; | O, 25.86. |
| Found: | C, 69.47; | H, 4.72; | O, 26.04. |

*7-(2-hydroxy-3-isopropylaminopropoxy)chrysin hydrochloride* — The hydrochloride was prepared as usual from the above epoxide and isopropylamine as a light yellow powder; m.p. 236—37°. (dec. *i*PrOH); 45% yield.

| | | | | |
|---|---|---|---|---|
| Anal. Calc'd. for $C_{21}H_{24}ClNO_5$: | C, 62.14; | H, 5.96; | Cl, 8.73; | N, 3.45. |
| Found: | C, 61.90; | H, 5.84; | Cl, 8.72; | N, 3.33. |

Example 18

Preparation of 7-(2-hydroxy-3-isopropylaminopropoxy)-2-methylisoflavone hydrochloride:

*7-(2,3-epoxypropoxy)-2-methylisoflavone* — Using Method A, the epoxide was synthesized from 7-hydro-2-methylisoflavone as white prisms.

*7-(2-hydroxy-3-isopropylaminopropoxy)-2-methylisoflavone hydrochloride* — The title compound was prepared as described in Method B from the above epoxide and isopropylamine as white prisms; m.p. 183—5° (*i*PrOH), in 40% yield.

| | | | | |
|---|---|---|---|---|
| Anal. Calc'd. for $C_{22}H_{26}ClNO_4$: | C, 65.42; | H, 6.49; | N, 3.47; | Cl, 8.78. |
| Found: | C, 65.25; | H, 6.74; | N, 3.39; | Cl, 8.67. |

Example 19

Preparation of 7-(3-N-methyl-N-isopropylamino-2-hydroxypropoxy)-2-methylisoflavone methiodide:

7-(2-hydroxy-3-isopropylaminopropoxy)-2-methylisoflavone (8.1 g, 0.021 moles) was added to a small amount of dry ether. The etheral solution under nitrogen was allowed to react with methyl iodide (9.0 ml) and stirred overnight. The solid formed was collected and washed with anhydrous ether. It was recrystallized twice from ethanol (abs.) to give 6.8 g (62% yield) of light yellow powder; m.p. 211—213°C.

| | | | | |
|---|---|---|---|---|
| Anal. Calc'd. for $C_{24}H_{30}NO_4I$: | C, 55.07; | H, 5.77; | I, 24.24; | N, 2.67. |
| Found: | C, 54.89; | H, 5.80; | I, 24.06; | N, 2.49. |

Example 20

Preparation of 7-(2-hydroxy-3-isopropylaminopropoxy)-2-trifluoromethylisoflavone hydrochloride:

*7-(2,3-epoxypropoxy)-2-trifluoromethylisoflavone* — The epoxide was prepared from 7-hydroxy-2-trifluoromethylisoflavone following Method A: m.p. 146—148° ($CH_2Cl_2$); yield, 61%.

*7-(2-hydroxy-3-isopropylaminopropoxy)-2-trifluoromethylisoflavone hydrochloride:* — Reaction of the corresponding epoxide with isopropylamine, as shown in Method B, afforded the title compound as white prisms, m.p. 220—222°C.

| | | | | | |
|---|---|---|---|---|---|
| Anal. Calc'd. for $C_{22}H_{23}ClFNO_4$: | C, 57.71; | H, 5.06; | Cl, 7.74; | F, 12.44; | N, 3.05. |
| Found: | C, 57.88; | H, 5.06; | Cl, 7.52; | F, 12.51; | N, 2.97. |

Example 21

Preparation of 7-(2-hydroxy-3-isopropylaminopropoxy)-2-isopropylisoflavone maleate:

*7-(2,3-epoxypropoxy)-2-isopropylisoflavone* — The epoxide was prepared from 7-hydroxy-2-isopropylisoflavone by Method A; m.p. 139—140°C ($CH_2Cl_2$); yield, 27%.

*7-(2-hydroxy-3-isopropylaminopropoxy)-2-isopropylisoflavone maleate* — The corresponding epoxide was reacted with isopropylamine followed by maleic acid to give the matleate as white prisms in 94% yield, m.p. 187—188°.

| | | | |
|---|---|---|---|
| Anal. Calc'd. for $C_{28}H_{33}NO_8$: | C, 65.74; | H, 6.50; | N, 2.73. |
| Found: | C, 65.77; | H, 6.27; | N, 2.66. |

Example 22

Preparation of 7-(3-allylamino-2-hydroxypropoxy)-2,3-diphenylchromone:

Using Method B, the reaction of 7-(2,3-epoxypropoxy)-2,3-diphenylchromone and allylamine afforded the desired amine as white needles, m.p. 119—(*i*PrOH); yield, 82%. Its hydrochloride salt had m.p. 140—142° (*i*PrOH/ph$CH_3$); containing water).

| | | | | |
|---|---|---|---|---|
| Anal. Calc'd. for $C_{27}H_{25}NO_4$ (the free base): | C, 75.85; | H, 5.89; | N, 3.27; | O, 14.97. |
| Found: | C, 75.87; | H, 5.95; | N, 3.14; | O, 15.04. |

Example 23

Preparation of 7-(3-cyclopentylamino-2-hydroxypropoxy)-2,3-diphenylchromone hydrochloride:

Using Method B, the reaction of 7-(2,3-epoxypropoxy)-2,3-diphenylchromone and cyclopentylamine gave the title amine as white crystals; m.p. 162.5—163° (*i*PrOH); yield 87%. Its hydrochloride salt was prepared as usual and melted at 155—156° (*i*PrOH).

| | | | | | |
|---|---|---|---|---|---|
| Anal. Calc'd. for $C_{29}H_{30}ClNO_4.1\frac{1}{2}H_2O$: | C, 67.11; | H, 6.41; | Cl, 6.83; | N, 2.70; | O, 16.95. |
| Found: | C, 67.04; | H, 5.90; | Cl, 6.70; | N, 2.66; | O, 17.04. |

Example 24

Preparation of 7-(3-sec-butylamine-2-hydroxypropoxy)-2,3-diphenylchromone

The title compound was synthesized according to Method B, in 98% yield, as white crystals, m.p. 139—139.5°.

| | | | |
|---|---|---|---|
| Anal. Calc'd. for $C_{28}H_{29}NO_4$: | C, 75.82; | H, 6.59; | N, 3.15. |
| Found: | C, 75.50; | H, 6.81; | N, 3.10. |

Example 25

Preparation of 7-(3-N-methyl-N-propylamino-2-hydroxypropoxy)-2,3-diphenylchromone maleate

Using Method B, the free base of the title compound was prepared from the corresponding epoxide and N-methylpropylamine. The obtained amine was dissolved in isopropyl alcohol and reacted with a solution of maleic acid in isopropyl alcohol. The white crystals formed were collected; m.p. 135—137°.

| | | | |
|---|---|---|---|
| Anal. Calc'd. for $C_{32}H_{33}NO_8$: | C, 68.68; | H, 5.94; | N, 2.50. |
| Found: | C, 68.88; | H, 5.98; | N, 2.26. |

Example 26

Preparation of 3-Methyl-7-(3-isopropylamino-2-hydroxypropoxy) flavone hydrochloride

*3-Methyl-7-(2,3-epoxypropoxy)flavone* — Using Method A, 3-methyl-7-hydroxyflavone was converted to its corresponding 7-epoxypropyl ether, m.p. 231—235° (crude); yield. 86%.

*3-Methyl-7-(2-hydroxy-3-isopropylaminopropoxy) flavone hydrochloride* — The amine salt was prepared as described in Method B to give a white solid, m.p. 141—144°.

| | | | | | |
|---|---|---|---|---|---|
| Anal. Calc'd. for $C_{22}H_{26}CLNO_4$: | C, 65.42; | H, 6.48; | Cl, 8.77; | N, 3.46; | O, 15.84. |
| Found: | C, 65.62; | H, 6.61; | Cl, 8.51; | N, 3.33; | O, 15.30. |

Antihypertensive activity was determined in the spontaneously hypertensive rat. Animals were dosed with compound or control vehicle following a control blood pressure (systolic) and heart rate determination made by means of an inflatable tail cuff. Measurements were made contained at 5 hours post dosing. Five animals per test group were employed. A 10% decrease in systolic blood pressure which was statistically different from both the control animals and the post dosage measurement is classified as minimal activity.

The following table gives minimal dosage required for minimal activity at 5 hours for the compounds of formula (1) and for a compound of the P. Da Re article, 3-methyl-7-(3-isopropylamino-2-hydroxypropoxy)flavone hydrochloride prepared using Method A and Method B, and shown in Example 26. The compounds in which $R_2$ and $R_3$ are both phenyl are surprisingly found to have distinctly superior advantages over those compounds in which $R_3$ is hydrogen, particularly for longer durations of activity. The compounds in which $R_1$ is cyclopropyl and *n*-propyl are also found to exhibit surprisingly superior activity.

Example 27

Pharmacology Data

| Example No. | $R_1$ | $R_2$ | $R_3$ | $R_4$ | Activity mg./kg. |
|---|---|---|---|---|---|
| 1 | *i*-propyl | phenyl | phenyl | hydrogen | 8 |
| 2 | *i*-propyl | phenyl | hydrogen | hydrogen | 17 |
| 3 | *i*-propyl | hydrogen | phenyl | hydrogen | 75 |
| 4 | *n*-propyl | phenyl | hydrogen | hydrogen | 12 |
| 5 | *i*-propyl | 2′Cl phenyl | hydrogen | hydrogen | 35 |
| 6 | *i*-propyl | phenyl | hydrogen | hydrogen | 75 |
| 7 | *i*-propyl | 4′Cl phenyl | hydrogen | hydrogen | 35 |
| 8 | *n*-propyl | phenyl | phenyl | hydrogen | 8 |
| 9 | cyclopropyl | phenyl | hydrogen | hydrogen | 8 |
| 10 | methyl | phenyl | hydrogen | hydrogen | 35 |
| 11 | ethyl | phenyl | hydrogen | hydrogen | 35 |
| 12 | *n*-butyl | phenyl | hydrogen | hydrogen | 17 |
| 13 | cyclohexyl | phenyl | hydrogen | hydrogen | 17 |
| 14 | cyclopropyl | phenyl | phenyl | hydrogen | 8 |
| 15 | hydrogen | phenyl | hydrogen | hydrogen | 75 |
| 16 | *n*-propyl | phenyl | benzyl | hydrogen | 75 |
| 17 | *i*-propyl | phenyl | hydrogen | hydroxyl | 75 |
| 18 | *i*-propyl | methyl | phenyl | hydrogen | 35 |
| 19 | $N_1$N-dimethyl-isopropylamino | methyl | phenyl | hydrogen | 75 |
| 20 | *i*-propyl | $CF_3$ | phenyl | hydrogen | 35 |
| 21 | *i*-propyl | *i*-propyl | phenyl | hydrogen | 35 |
| 22 | allyl | phenyl | phenyl | hydrogen | 75 |
| 23 | cyclopentyl | phenyl | phenyl | hydrogen | 75 |
| 24 | sec-butyl | phenyl | phenyl | hydrogen | 75 |
| 25 | N-methyl-N propyl | phenyl | phenyl | hydrogen | 75 |
| 26 | *i*-propyl | phenyl | methyl | hydrogen | 75 |

These compounds do not exhibit beta-blocking activity when determined as antagonism of isoproterenol-induced beta stimulation of isolated rat heart or inhibition of isoproterenol-induced effects on the cardiovascular system of the anesthetized rat.

**Claims for the Contracting States: BE CH DE FR GB IT LI LU NL SE**

1. A chromone of the formula

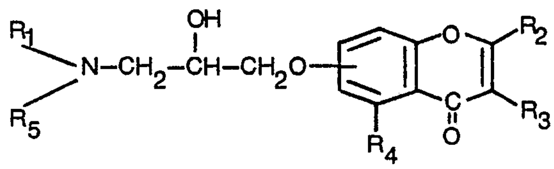

or a pharmaceutically acceptable salt thereof wherein, the $R_1R_5N—CH_2—CH(OH)CH_2O$ group is in the 6 or 7 position of the chromone nucleus; $R_1$ is H, primary or secondary alkyl of 1 to 4 carbon atoms, or cycloalkyl of 3 to 6 carbon atoms; $R_2$ is H, lower alkyl comprising 1 to 4 carbon atoms, $CF_3$, phenyl, o-chlorophenyl or p-chlorophenyl, $R_3$ is H or phenyl or benzyl; $R_4$ is hydrogen or hydroxyl; $R_5$ is hydrogen or methyl; provided, however, that at least one of $R_2$ or $R_3$ is phenyl or substituted phenyl, and that $R_5$ is methyl only if $R_1$ is isopropyl, $R_2$ is methyl, $R_3$ is phenyl and $R_4$ is hydrogen or $R_1$ is propyl, $R_2$ is phenyl, $R_3$ is phenyl and $R_4$ is hydrogen for use as an antihypertensive.

2. A chromone of the formula

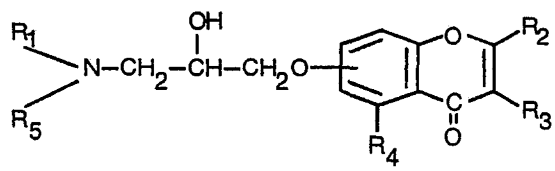

or a pharmaceutically acceptable salt thereof wherein the $R_1R_5N—CH_2—CH(OH)CH_2O$ group is in the 6 or 7 position of the chromone nucleus; $R_1$ is H, primary or secondary alkyl of 1 to 4 carbon atoms, or cycloalkyl of 3 to 6 carbon atoms; $R_2$ is H, lower alkyl comprising 1 to 4 carbon atoms, $CF_3$, phenyl, o-chlorophenyl or p-chlorophenyl; $R_3$ is H or phenyl or benzyl; $R_4$ is hydrogen or hydroxyl; $R_5$ is hydrogen or methyl; provided, however, that at least one of $R_2$ or $R_3$ is phenyl or substituted phenyl and that $R_5$ is methyl only if $R_1$ is isopropyl, $R_2$ is methyl, $R_3$ is phenyl and $R_4$ is hydrogen or $R_1$ is propyl, $R_2$ is phenyl, $R_3$ is phenyl and $R_4$ is hydrogen; and provided further that when $R_2$ is phenyl, $R_3$ is hydrogen and $R_4$ is hydrogen then $R_1$ is not isopropyl.

3. The chromone of claim 1 or 2, wherein the $R_1R_5N—CH_2—CH(OH)CH_2O$ group is in the 7 position.

4. The chromone of claim 1 or 2, wherein the $R_1R_5N—CH_2—CH(OH)CH_2O$ group is in the 7 position of the chromone nucleus; $R_1$ is i-propyl, n-propyl or cyclopropyl; $R_2$ and $R_3$ are phenyl; $R_4$ is hydrogen; and $R_5$ is hydrogen.

5. The chromone of claim 3, wherein the chromone is 7-(3-n-propylamino-2-hydroxypropoxy)-2,3-diphenylchromone hydrochloride.

6. The chromone of claim 3, 7-(2-hydroxy-3-isopropylaminopropoxy)-2,3-diphenylchromone hydrochloride.

7. The chromone of claim 3, 7-(3-cyclopropylamino-2-hydroxypropoxy)-2,3-diphenylchromone.

8. The chromone of claim 1 or 2, 7-(2-hydroxy-3-isopropylaminopropoxy)-3-phenylchromone hydrochloride.

9. The chromone of claim 1 or 2, 7-(2-hydroxy-3-isopropylaminopropoxy)-2-methylisoflavone hydrochloride.

10. The chromone of claim 1 or 2, 7-(3-N,N-dimethyl-N-isopropylamino-2-hydroxypropoxy)-2-methylisoflavone methiodide.

11. The chromone of claim 1 or 2, 7-(2-hydroxy-3-isopropylaminopropoxy)-2-trifluoromethylisoflavone hydrochloride.

12. The chromone of claim 1 or 2, 7-(2-hydroxy-3-isopropylaminopropoxy)-2-isopropylisoflavone maleate.

13. The chromone of claim 1 or 2 having the formula

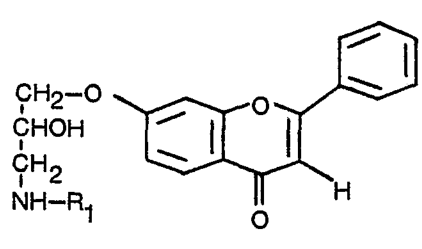

or a pharmaceutically acceptable salt thereof wherein $R_1$ is n-propyl or cyclopropyl.

14. The chromone of claim 13, 7-(3-cyclopropylamino-2-hydroxypropoxy)flavone hydrochloride.

15. The chromone of claim 13, 7-(3-n-propylamino-2-hydroxypropoxy)flavone hydrochloride.

16. The maleate salts of the chromones of claim 13.

17. A pharmaceutical composition comprising (a) one or more of the chromones of claims 2 to 16, and (b) a pharmaceutically acceptable carrier.

**Claims for the Contracting State: AT**

1. A process for the production of a pharmaceutical composition for use as an antihypertensive, comprising mixing a chromone of the formula

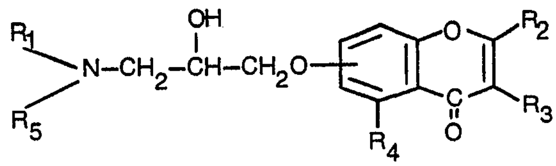

or a pharmaceutically acceptable salt thereof wherein, the $R_1R_5N—CH_2—CH(OH)CH_2O$ group is in the 6 or 7 position of the chromone nucleus; $R_1$ is H, primary or secondary alkyl of 1 to 4 carbon atoms, or cycloalkyl of 3 to 6 carbon atoms; $R_2$ is H, lower alkyl comprising 1 to 4 carbon atoms, $CF_3$, phenyl, o-chlorophenyl or p-chlorophenyl, $R_3$ is H or phenyl or benzyl; $R_4$ is hydrogen or hydroxyl; $R_5$ is hydrogen or methyl; provided, however, that at least one of $R_2$ or $R_3$ is phenyl or substituted phenyl, and that $R_5$ is methyl only if $R_1$ is isopropyl, $R_2$ is methyl, $R_3$ is phenyl and $R_4$ is hydrogen or $R_1$ is propyl, $R_2$ is phenyl, $R_3$ is phenyl and $R_4$ is hydrogen; and a pharmaceutically acceptable carrier.

2. A process for the production of a chromone of the formula

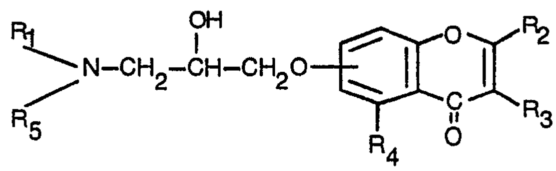

or a pharmaceutically acceptable salt thereof wherein the $R_1R_5N—CH_2—CH(OH)CH_2O$ group is in the 6 or 7 position of the chromone nucleus; $R_1$ is H, primary or secondary alkyl of 1 to 4 carbon atoms, or cycloalkyl of 3 to 6 carbon atoms; $R_2$ is H, lower alkyl comprising 1 to 4 carbon atoms, $CF_3$, phenyl, o-chlorophenyl or p-chlorophenyl; $R_3$ is H or phenyl or benzyl; $R_4$ is hydrogen or hydroxyl; $R_5$ is hydrogen or methyl; provided, however, that at least one of $R_2$ or $R_3$ is phenyl or substituted phenyl and that $R_5$ is methyl only if $R_1$ is isopropyl, $R_2$ is methyl, $R_3$ is phenyl and $R_4$ is hydrogen or $R_1$ is propyl, $R_2$ is phenyl, $R_3$ is phenyl and $R_4$ is hydrogen; and provided further that when $R_2$ is phenyl, $R_3$ is hydrogen and $R_4$ is hydrogen then $R_1$ is not isopropyl; characterized by reacting epichlorohydrin or epibromohydrin with a compound of the formula

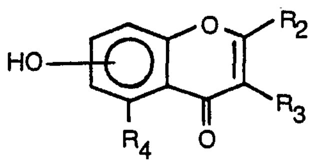

wherein $R_2$, $R_3$ and $R_4$ are the same as defined above and the hydroxyl group is substituted on the 6 or 7 position, in the presence of a solvent and a base to give an epoxide of the above formula where the hydroxyl group is replaced by

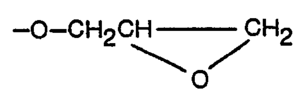

and reacting this epoxide with an amine of the formula $R_1R_5NH$, where $R_1$ and $R_5$ are as above defined, in an alcoholic solvent at elevated temperature and thereafter optionally converting the obtained compound into a pharmaceutically acceptable salt thereof.

3. The process of claim 1 or 2, wherein the $R_1R_5N—CH_2—CH(OH)CH_2O$ group is in the 7 position.

4. The process of claim 1 or 2, wherein the $R_1R_5N—CH_2—CH(OH)CH_2O$ group is in the 7 position of the chromone nucleus;

$R_1$ is i-propyl, n-propyl or cyclopropyl; $R_2$ and $R_3$ are phenyl; $R_4$ is hydrogen; and $R_5$ is hydrogen.

5. The process of claim 3, wherein the chromone is 7-(3-n-propylamino-2-hydroxypropoxy)-2,3-diphenylchromone hydrochloride.

6. The process of claim 3, wherein the chromone is 7-(2-hydroxy-3-isopropylaminopropoxy)-2,3-diphenylchromone hydrochloride.

7. The process of claim 3, wherein the chromone is 7-(3-cyclopropylamino-2-hydroxypropoxy)-2,3-diphenylchromone.

8. The process of claim 1 or 2, wherein the chromone is 7-(2-hydroxy-3-isopropylaminopropoxy)-3-phenylchromone hydrochloride.

9. The process of claim 1 or 2, wherein the chromone is 7-(2-hydroxy-3-isopropylaminopropoxy)-2-methylisoflavone hydrochloride.

10. The process of claim 1 or 2, wherein the chromone is 7-(3-$N_1N$-dimethyl-N-isopropylamino-2-hydroxypropoxy)-2-methylisoflavone methiodide.

11. The process of claim 1 or 2, wherein the chromone is 7-(2-hydroxy-3-isopropylaminopropoxy)-2-trifluoromethylisoflavone hydrochloride.

12. The process of claim 2 wherein $R_1$ is n-propyl and $R_5$ is hydrogen.

13. The process of claim 3 wherein the salt formed is the hydrochloride.

14. The process of claim 3 wherein the salt formed is the maleate.

15. Process for the production of the chromone of claim 2 having the formula (I)

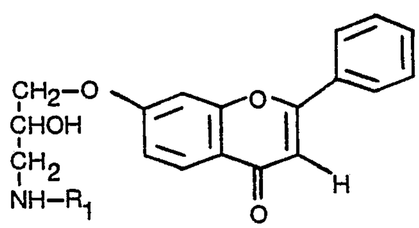

or a pharmaceutically acceptable salt thereof wherein $R_1$ is n-propyl or cyclopropyl, characterized by reacting epichlorohydrin or epibromohydrin with a compound of the formula

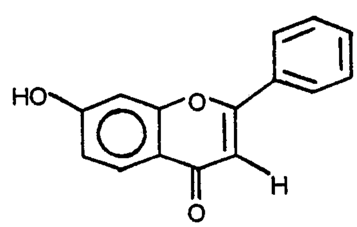

in the presence of a solvent and a base to give an epoxide of the above formula where the hydroxyl group is replaced by

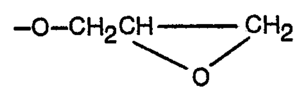

and reacting this epoxide with an amine having the formula $R_1NH_2$ where $R_1$ is as above defined, in an alcoholic solvent at elevated temperature to afford a product of formula (I) and optionally converting the amine into its pharmaceutically acceptable salt.

16. The process of claim 15, wherein 7-(3-cyclopropylamino-2-hydroxypropoxy)flavone hydrochloride is prepared.

17. The process of claim 15, wherein 7-(3-n-propylamino-2-hydroxypropoxy)flavone hydrochloride is prepared.

18. The process of claim 15, wherein the maleate salts of the chromones are prepared.

**Patentansprüche für die Vertragsstaaten: BE CH DE FR GB IT LI LU NL SE**

1. Chromon der Formel:

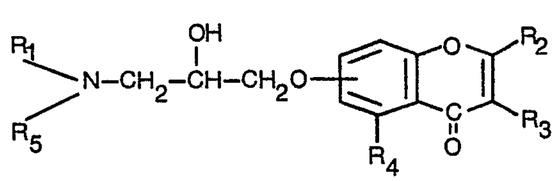

oder ein pharmazeutisch annehmbares Salz davon, worin die $R_1R_5N—CH_2—CH(OH)CH_2O$-Gruppe sich in 6- oder 7-Position des Chromonkerns befindet, $R_1$ für H, primäres oder sekundäres Alkyl mit 1 bis 4 Kohlenstoffatomen oder Cycloalkyl mit 3 bis 6 Kohlenstoffatomen steht, $R_2$ für H, Niedrigalkyl mit 1 bis 4 Kohlenstoffatomen, $CF_3$, Phenyl, o-Chlorphenyl oder p-Chlorphenyl steht, $R_3$ für H oder Phenyl oder Benzyl steht, $R_4$ für Wasserstoff oder Hydroxyl steht, $R_5$ für Wasserstoff oder Methyl steht, jedoch mit der Maßgabe, daß mindestens eine der Gruppen $R_2$ oder $R_3$ Phenyl oder substituiertes Phenyl ist, und daß $R_5$ nur dann Methyl ist, wenn $R_1$ Isopropyl ist, $R_2$ Methyl ist, $R_3$ Phenyl ist und $R_4$ Wasserstoff ist, oder wenn $R_1$ Propyl ist, $R_2$ Phenyl ist, $R_3$ Phenyl ist und $R_4$ Wasserstoff ist, zur Verwendung als antihypertensives Mittel.

2. Chromon der Formel:

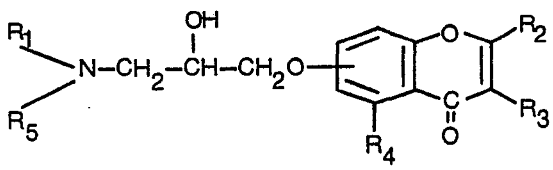

oder ein pharmazeutisch annehmbares Salz davon, worin die $R_1R_5N—CH_2—CH(OH)CH_2O$-Gruppe sich in 6- oder 7-Position des Chromonkerns befindet, $R_1$ für H, primäres oder sekundäres Alkyl mit 1 bis 4 Kohlenstoffatomen oder Cycloalkyl mit 3 bis 6 Kohlenstoffatomen steht, $R_2$ für H, Niedrigalkyl mit 1 bis 4 Kohlenstoffatomen, $CF_3$, Phenyl, o-Chlorphenyl oder p-Chlorphenyl steht, $R_3$ für H oder Phenyl oder Benzyl steht, $R_4$ für Wasserstoff oder Hydroxyl steht, $R_5$ für Wasserstoff oder Methyl steht, jedoch mit der Maßgabe, daß mindestens eine der Gruppen $R_2$ oder $R_3$ Phenyl oder substituiertes Phenyl ist und daß $R_5$ nur dann Methyl ist, wenn $R_1$ Isopropyl ist, $R_2$ Methyl ist, $R_3$ Phenyl ist und $R_4$ Wasserstoff ist oder wenn $R_1$ Propyl ist, $R_2$ Phenyl ist, $R_3$ Phenyl ist und $R_4$ Wasserstoff ist, und mit der weiteren Maßgabe, daß, wenn $R_2$ Phenyl, $R_3$ Wasserstoff und $R_4$ Wasserstoff sind, dann $R_1$ nicht Isopropyl ist.

3. Chromon nach Anspruch 1 oder 2, dadurch gekennzeichnet, daß sich die $R_1R_5N—CH_2—CH(OH)CH_2O$-Gruppe in 7-Position befindet.

4. Chromon nach Anspruch 1 oder 2, dadurch gekennzeichnet, daß sich die $R_1R_5N—CH_2—CH(OH)CH_2O$-Gruppe in 7-Position des Chromonkerns befindet, daß $R_1$ für i-Propyl, n-Propyl oder Cyclopropyl steht, $R_2$ und $R_3$ für Phenyl stehen, $R_4$ für Wasserstoff und $R_5$ für Wasserstoff stehen.

5. Chromon nach Anspruch 3, dadurch gekennzeichnet, daß das Chromon 7-(3-n-Propylamino-2-hydroxypropoxy)-2,3-diphenylchromon-hydrochlorid ist.

6. Chromon nach Anspruch 3, nämlich 7-(2-Hydroxy-3-isopropylaminopropoxy)-2,3-diphenylchromonhydrochlorid.

7. Chromon nach Anspruch 3, nämlich 7-(3-Cyclopropylamino-2-hydroxypropoxy)-2,3-diphenylchromon.

8. Chromon nach Anspruch 1 oder 2, nämlich 7-(2-Hydroxy-3-isopropylaminopropoxy)-3-phenylchromonhydrochlorid.

9. Chromon nach Anspruch 1 oder 2, nämlich 7-(2-Hydroxy-3-isopropylaminopropoxy)-2-methylisoflavonhydrochlorid.

10. Chromon nach Anspruch 1 oder 2, nämlich 7-(3-N,N-Dimethyl-N-isopropylamino-2-hydroxypropoxy)-2-methylisoflavonmethjodid.

11. Chromon nach Anspruch 1 oder 2, nämlich 7-(2-Hydroxy-2-isopropylaminopropoxy)-2-trifluormethylisoflavonhydrochlorid.

12. Chromon nach Anspruch 1 oder 2, nämlich 7-(2-Hydroxy-3-isopropylaminopropoxy)-2-isopropylisoflavonmaleat.

13. Chromon nach Anspruch 1 oder 2 mit der Formel:

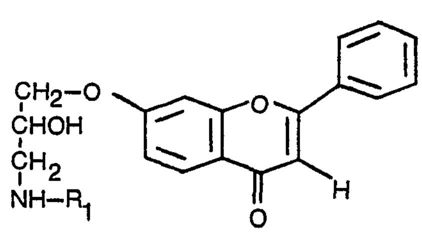

oder ein pharmazeutisch annehmbares Salz davon, worin $R_1$ für n-Propyl oder Cyclopropyl steht.

14. Chromon nach Anspruch 13, nämlich 7-(3-Cyclopropylamino-2-hydroxypropoxy)-flavonhydrochlorid.

15. Chromon nach Anspruch 13, nämlich 7-(3-n-Propylamino-2-hydroxypropoxy)-flavon-hydrochlorid.

16. Maleatsalze der Chromone nach Anspruch 13.

17. Arzneimittel, dadurch gekennzeichnet, daß es (a) ein oder mehrere der Chromone der Ansprüche 2 bis 16 und (b) einen pharmazeutisch annehmbaren Träger enthält.

**Patentansprüche für den Vertragsstaat: AT**

1. Verfahren zur Herstellung eines Arzneimittels zur Verwendung als antihypertensives Mittel, dadurch gekennzeichnet, daß man ein Chromon der Formel:

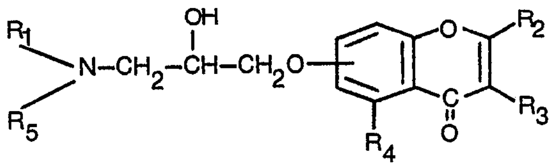

oder ein pharmazeutisch annehmbares Salz davon, worin die $R_1R_5N—CH_2—CH(OH)CH_2O$-Gruppe sich in 6- oder 7-Position des Chromonkerns befindet, $R_1$ für H, primäres oder sekundäres Alkyl mit 1 bis 4 Kohlenstoffatomen oder Cycloalkyl mit 3 bis 6 Kohlenstoffatomen steht, $R_2$ für H, Niedrigalkyl mit 1 bis 4 Kohlenstoffatomen, $CF_3$, Phenyl, o-Chlorphenyl oder p-Chlorphenyl steht, $R_3$ für H oder Phenyl oder Benzyl steht, $R_4$ für Wasserstoff oder Hydroxyl steht, $R_5$ für Wasserstoff oder Methyl steht, jedoch mit der Maßgabe, daß mindestens eine der Gruppen $R_2$ oder $R_3$ Phenyl oder substituiertes Phenyl ist, und daß $R_5$ nur dann Methyl ist, wenn $R_1$ Isopropyl ist, $R_2$ Methyl ist, $R_3$ Phenyl ist und $R_4$ Wasserstoff ist, oder wenn $R_1$ Propyl ist, $R_2$ Phenyl ist, $R_3$ Phenyl ist und $R_4$ Wasserstoff ist, und einen pharmazeutisch annehmbaren Träger vermischt.

2. Verfahren zur Herstellung eines Chromons der Formel:

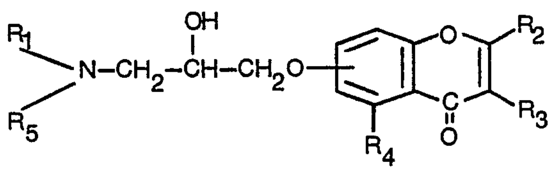

oder eines pharmazeutisch annehmbaren Salzes davon, worin die $R_1R_5N—CH_2—CH(OH)CH_2O$-Gruppe sich in 6- oder 7-Position des Chromonkerns befindet, $R_1$ für H, primäres oder sekundäres Alkyl mit 1 bis 4 Kohlenstoffatomen oder Cycloalkyl mit 3 bis 6 Kohlenstoffatomen steht, $R_2$ für H, Niedrigalkyl mit 1 bis 4 Kohlenstoffatomen, $CF_3$, Phenyl, o-Chlorphenyl oder p-Chlorphenyl steht, $R_3$ für H oder Phenyl oder Benzyl steht, $R_4$ für Wasserstoff oder Hydroxyl steht, $R_5$ für Wasserstoff oder Methyl steht, jedoch mit der Maßgabe, daß mindestens eine der Gruppen $R_2$ oder $R_3$ Phenyl oder substituiertes Phenyl ist und daß $R_5$ nur dann Methyl ist, wenn $R_1$ Isopropyl ist, $R_2$ Methyl ist, $R_3$ Phenyl ist und $R_4$ Wasserstoff ist oder wenn $R_1$ Propyl ist, $R_2$ Phenyl ist, $R_3$ Phenyl ist und $R_4$ Wasserstoff ist, und mit der weiteren Maßgabe, daß, wenn $R_2$ Phenyl, $R_3$ Wasserstoff und $R_4$ Wasserstoff sind, dann $R_1$ nicht Isopropyl ist, dadurch gekennzeichnet, daß man Epichlorhydrin oder Epibromhydrin mit einer Verbindung der Formel:

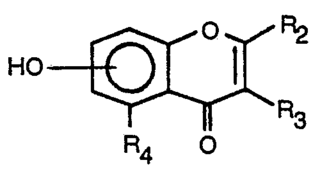

worin $R_2$, $R_3$ und $R_4$ wie oben definiert sind und die Hydroxylgruppe in 6- oder 7-Position substituiert ist, in Gegenwart eines Lösungsmittels und einer Base umsetzt, um ein Epoxid der obigen Formel zu erhalten, bei dem die Hydroxylgruppe durch

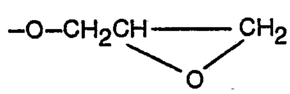

ersetzt ist, und daß man dieses Epoxid mit einem Amin der Formel $R_1R_5NH$, worin $R_1$ und $R_5$ wie oben definiert sind, in einem alkoholischen Lösungsmittel bei erhöhter Temperatur umsetzt und danach gegebenenfalls die erhaltene Verbindung in ein pharmazeutisch annehmbares Salz davon umwandelt.

3. Verfahren nach Anspruch 1 oder 2, dadurch gekennzeichnet, daß sich die $R_1R_5N—CH_2—CH(OH)CH_2O$-Gruppe in 7-Position befindet.

4. Verfahren nach Anspruch 1 oder 2, dadurch gekennzeichnet, daß sich die $R_1R_5N—CH_2—CH(OH)CH_2O$-Gruppe in 7-Position des Chromonkerns befindet, daß $R_1$ für Isopropyl, n-Propyl oder Cyclopropyl steht, $R_2$ und $R_3$ für Phenyl stehen, $R_4$ für Wasserstoff steht und $R_5$ für Wassertoff steht.

5. Verfahren nach Anspruch 3, dadurch gekennzeichnet, daß das Chromon 7-(3-n-Propylamino-2-hydroxypropoxy)-2,3-diphenylchromon-hydrochlorid ist.

6. Verfahren nach Anspruch 3, dadurch gekennzeichnet, daß das Chromon 7-(2-Hydroxy-3-isopropylaminopropoxy)-2,3-diphenylchromon-hydrochlorid ist.

7. Verfahren nach Anspruch 3, dadurch gekennzeichnet, daß das Chromon 7-(3-Cyclopropylamino-2-hydroxypropoxy)-2,3-diphenylchromon ist.

8. Verfahren nach Anspruch 1 oder 2, dadurch gekennzeichnet, daß das Chromon 7-(2-Hydroxy-3-isopropylaminopropoxy)-3-phenylchromon-hydrochlorid ist.

9. Verfahren nach Anspruch 1 oder 2, dadurch gekennzeichnet, daß das Chromon 7-(2-Hydroxy-3-isopropylaminopropoxy)-2-methylisoflavon-hydrochlorid ist.

10. Verfahren nach Anspruch 1 oder 2, dadurch gekennzeichnet, daß das Chromon 7-(3-$N_1$N-Dimethyl-N-isopropylamino-2-hydroxypropoxy)-2-methylisoflavon-methjodid ist.

11. Verfahren nach Anspruch 1 oder 2, dadurch gekennzeichnet, daß das Chromon 7-(2-Hydroxy-3-isopropylaminopropoxy)-2-trifluormethylisoflavon-hydrochlorid ist.

12. Verfahren nach Anspruch 2, dadurch gekennzeichnet, daß $R_1$ für n-Propyl steht und daß $R_5$ für Wasserstoff steht.

13. Verfahren nach Anspruch 3, dadurch gekennzeichnet, daß das gebildete Salz das Hydrochlorid ist.

14. Verfahren nach Anspruch 3, dadurch gekennzeichnet, daß das gebildete Salz das Maleat ist.

15. Verfahren zur Herstellung des Chromons nach Anspruch 2 mit der Formel (I):

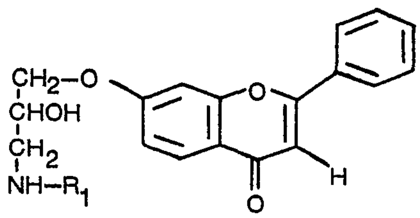

oder eines pharmazeutisch annehmbaren Salzes davon, wobei $R_1$ für n-Propyl oder Cyclopropyl steht, dadurch gekennzeichnet, daß man Epichlorhydrin oder Epibromhydrin mit einer Verbindung der Formel:

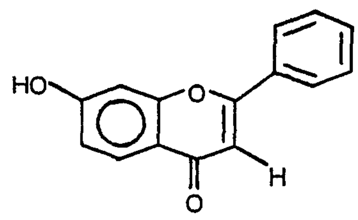

in Gegenwart eines Lösungsmittels und einer Base umsetzt, um ein Epoxid der obigen Formel zu erhalten, bei der die Hydroxylgruppe durch

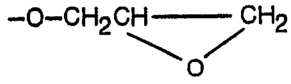

ersetzt ist, und daß man dieses Epoxid mit einem Amin mit der Formel $R_1NH_2$, worin $R_1$ wie oben definiert ist, in einem alkoholischen Lösungsmittel bei erhöhter Temperatur umsetzt, um ein Produkt der Formel (I) zu erhalten, und daß man gegebenenfalls das Amin in sein pharmazeutisch annehmbares Salz umwandelt.

16. Verfahren nach Anspruch 15, dadurch gekennzeichnet, daß man das 7-(3-Cyclopropylamino-2-hydroxypropoxy)-flavon-hydrochlorid herstellt.

17. Verfahren nach Anspruch 15, dadurch gekennzeichnet, daß man das 7-(3-n-Propylamino-2-hydroxypropoxy)-flavon-hydrochlorid herstellt.

18. Verfahren nach Anspruch 15, dadurch gekennzeichnet, daß man die Maleatsalze der Chromone herstellt.

**Revendications pour les Etats contractants: BE CH DE FR GB IT LI LU NL SE**

1. Chromone de formule

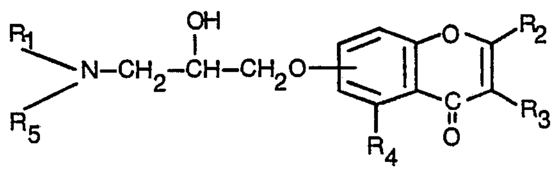

ou un sel pharmaceutiquement acceptable de ce composé, formule dans laquelle le groupe $R_1R_5N—CH_2—CH(OH)CH_2O$ est en position 6 ou 7 du noyau de chromone;

$R_1$ représente H, un groupe alkyle primaire ou secondaire ayant 1 à 4 atomes de carbone ou un groupe cycloalkyle de 3 à 6 atomes de carbone;

$R_2$ représente H, un groupe alkyle inférieur comprenant 1 à 4 atomes de carbone, $CF_3$, phényle, o-chlorophényle ou p-chlorophényle,

$R_3$ représente H ou un groupe phényle ou benzyle;

$R_4$ est l'hydrogène ou un groupe hydroxyle;

$R_5$ est l'hydrogène ou un groupe méthyle; sous réserve, toutefois, qu'au moins l'un de $R_2$ et $R_3$ soit un groupe phényle ou un groupe phényle substitué, et que $R_5$ ne soit un groupe méthyle que si $R_1$ est un groupe isopropyle, $R_2$ est un groupe méthyle, $R_3$ est un groupe phényle et $R_4$ est l'hydrogène ou si $R_1$ est un groupe propyle, $R_2$ est un groupe phényle, $R_3$ est un groupe phényle et $R_4$ est l'hydrogène; destiné à être utilisé comme antihypertenseur.

2. Chromone de formule

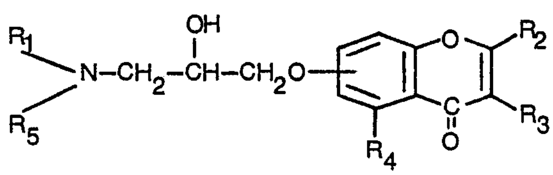

ou un sel pharmaceutiquement acceptable de ce composé, formule dans laquelle le groupe $R_1R_5N—CH_2—CH(OH)CH_2O$ est en position 6 ou 7 du noyau de chromone;

$R_1$ représente H, un groupe alkyle primaire ou secondaire de 1 à 4 atomes de carbone ou cycloalkyle de 3 à 6 atomes de carbone;

$R_2$ représente H, un groupe alkyle inférieur comprenant 1 à 4 atomes de carbone, $CF_3$, phényle, o-chlorophényle ou p-chlorophényle;

$R_3$ représente H ou un groupe phényle ou benzyle;

$R_4$ représente l'hydrogène ou un groupe hydroxyle;

$R_5$ représente l'hydrogène ou un groupe méthyle;

sous réserve, toutefois, qu'au moins l'un de $R_2$ et $R_3$ soit un groupe phényle ou phényle substitué et que $R_5$ ne soit un groupe méthyle que si $R_1$ est un groupe isopropyle, $R_2$ est un groupe méthyle, $R_3$ est un groupe phényle et $R_4$ est l'hydrogène, ou si $R_1$ est un groupe propyle, $R_2$ est un groupe phényle, $R_3$ est un groupe phényle et $R_4$ est un atome d'hydrogène; et sous réserve en outre que lorsque $R_2$ est un groupe phényle, $R_3$ est l'hydrogène et $R_4$ est l'hydrogène, $R_1$ ne soit pas un groupe isopropyle.

3. Chromone suivant la revendication 1 ou 2, dans laquelle le groupe $R_1R_5N—CH_2—CH(OH)CH_2O$ est en position 7.

4. Chromone suivant la revendication 1 ou 2, dans laquelle le groupe $R_1R_5N—CH_2—CH(OH)CH_2O$ est en position 7 du noyau de chromone;

$R_1$ est un groupe isopropyle, n-propyle ou cyclopropyle;

$R_2$ et $R_3$ représentent un groupe phényle;

$R_4$ est l'hydrogène; et

$R_5$ est l'hydrogène.

5. Chromone suivant la revendication 3, qui est le chlorhydrate de 7-(3-n-propylamino-2-hydroxypropoxy)-2,3-diphénylchromone.

6. Chromone suivant la revendication 3, qui est le chlorhydrate de 7-(2-hydroxy-3-isopropylaminopropoxy)-2,3-diphénylchromone.

7. Chromone suivant la revendication 3, qui est la 7-(3-cyclopropylamino-2-hydroxypropoxy)-2,3-diphénylchromone.

8. Chromone suivant la revendication 1 ou 2, qui est le chlorhydrate de 7-(2-hydroxy-3-isopropylaminopropoxy)-3-phénylchromone.

9. Chromone suivant la revendication 1 ou 2, qui est le chlorhydrate de 7-(2-hydroxy-3-isopropylaminopropoxy)-2-méthylisoflavone.

10. Chromone suivant la revendication 1 ou 2, qui est le méthiodure de 7-(3-$N_1$N-diméthyl-N-isopropylamino-2-hydroxypropoxy)-2-méthylisoflavone.

11. Chromone suivant la revendication 1 ou 2, qui est le chlorhydrate de 7-(2-hydroxy-3-isopropylaminopropoxy)-2-trifluorométhylisoflavone.

12. Chromone suivant la revendication 1 ou 2, qui est le maléate de 7-(2-hydroxy-3-isopropylaminopropoxy)-2-isopropylisoflavone.

13. Chromone suivant la revendication 1 ou 2, de formule

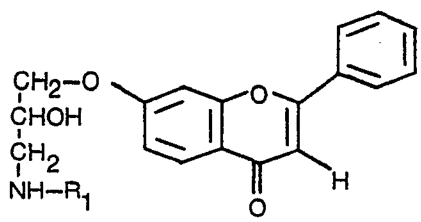

ou un sel pharmaceutiquement acceptable de cette chromone, dans la formule de laquelle $R_1$ est le groupe n-propyle ou cyclopropyle.

14. Chromone suivant la revendication 13, qui est le chlorhydrate de 7-(3-cyclopropylamino-2-hydroxypropoxy)flavone.

15. Chromone suivant la revendication 13, qui est le chlorhydrate de 7-(3-n-propylamino-2-hydroxypropoxy)flavone.

16. Les maléates des chromones suivant la revendication 13.

17. Composition pharmaceutique, comprenant (a) une ou plusieurs des chromones des revendications 2 à 16 et (b) un support pharmaceutiquement acceptables.

**Revendications pour l'Etat contractant: AT**

1. Procédé de préparation d'une composition pharmaceutique destinée à être utilisée comme antihypertenseur, consistant à mélanger une chromone de formule

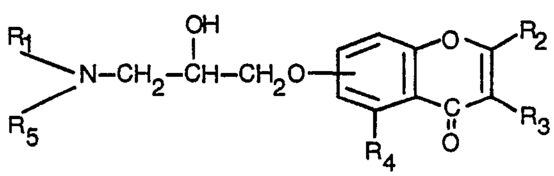

ou un sel pharmaceutiquement acceptable de ce composé, formule dans laquelle le groupe $R_1R_5N—CH_2—CH(OH)CH_2O$ est en position 6 ou 7 du noyau de chromone;

$R_1$ représente H, un groupe alkyle primaire ou secondaire ayant 1 à 4 atomes de carbone ou un groupe cycloalkyle de 3 à 6 atomes de carbone;

$R_2$ représente H, un groupe alkyle inférieur comprenant 1 à 4 atomes de carbone, $CF_3$, phényle, o-chlorophényle ou p-chlorophényle,

$R_3$ représente H ou un groupe phényle ou benzyle;

$R_4$ est l'hydrogène ou un groupe hydroxyle;

$R_5$ est l'hydrogène ou un groupe méthyle; sous réserve, toutefois, qu'au moins l'un de $R_2$ et $R_3$ soit un groupe phényle ou un groupe phényle substitué, et que $R_5$ ne soit un groupe méthyle que si $R_1$ est un groupe isopropyle, $R_2$ est un groupe méthyle, $R_3$ est un groupe phényle et $R_4$ est l'hydrogène ou si $R_1$ est un groupe propyle, $R_2$ est un groupe phényle, $R_3$ est un groupe phényle et $R_4$ est l'hydrogène; et un support pharmaceutiquement acceptable.

2. Procédé de production d'une chromone de formule

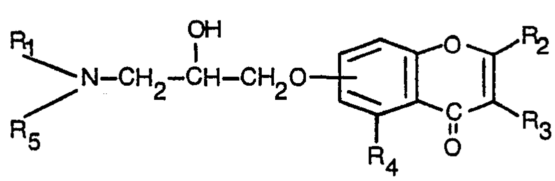

ou d'un sel pharmaceutiquement acceptable de ce composé, formule dans laquelle le groupe $R_1R_5N—CH_2—CH(OH)CH_2O$ est en position 6 ou 7 du noyau de chromone;

$R_1$ représente H, un groupe alkyle primaire ou secondaire de 1 à 4 atomes de carbone ou cycloalkyle de 3 à 6 atomes de carbone;

$R_2$ représente H, un groupe alkyle inférieur comprenant 1 à 4 atomes de carbone, $CF_3$, phényle, o-chlorophényle ou p-chlorophényle;

$R_3$ représente H ou un groupe phényle ou benzyle;

$R_4$ représente l'hydrogène ou un groupe hydroxyle;

$R_5$ représente l'hydrogène ou un groupe méthyle;

sous réserve, toutefois, qu'au moins l'un de $R_2$ et $R_3$ soit un groupe phényle ou phényle substitué et que $R_5$ ne soit un groupe méthyle que si $R_1$ est un groupe isopropyle, $R_2$ est un groupe méthyle, $R_3$ est un groupe phényle et $R_4$ est l'hydrogène, ou si $R_1$ est un groupe propyle, $R_2$ est un groupe phényle, $R_3$ est un groupe phényle et $R_4$ est un atome d'hydrogène; et sous réserve en outre que lorsque $R_2$ est un groupe phényle, $R_3$ est l'hydrogène et $R_4$ est l'hydrogène, $R_1$ ne soit pas un groupe isopropyle; caractérisé par la réaction de l'épichlorhydrine ou de l'épibromhydrine avec un composé de formule

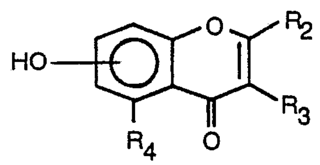

dans laquelle $R_2$, $R_3$ et $R_4$ sont tels que définis ci-dessus et le groupe hydroxyle est attaché en position 6 ou 7, en présence d'un solvant et d'une base pour former un époxyde de la formule ci-dessus dans laquelle le groupe hydroxyle est remplacé par un groupe

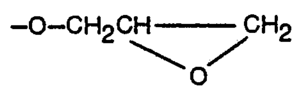

et la réaction de cet époxyde avec une amine de formule $R_1R_5NH$, dans laquelle $R_1$ et $R_5$ sont tels que définis ci-dessus, dans un solvant alcoolique à une température élevée, puis, à titre facultatif, la transformation du composé obtenu en un sel pharmaceutiquement acceptable de ce composé.

3. Procédé suivant la revendication 1 ou 2, dans lequel le groupe $R_1R_5N—CH_2—CH(OH)CH_2O$ est en position 7.

4. Procédé suivant la revendication 1 ou 2, dans lequel le groupe $R_1R_5N—CH_2—CH(OH)CH_2O$ est en position 7 du noyau de chromone;

$R_1$ est un groupe isopropyle, n-propyle ou cyclopropyle;

$R_2$ et $R_3$ sont des groupes phényle;

$R_4$ est l'hydrogène; et

$R_5$ est l'hydrogène.

5. Procédé suivant la revendication 3, dans lequel la chromone est le chlorhydrate de 7-(3-n-propylamino-2-hydroxypropoxy)-2,3-diphénylchromone.

6. Procédé suivant la revendication 3, dans lequel la chromone est le chlorhydrate de 7-(2-hydroxy-3-isopropylaminopropoxy)-2,3-diphénylchromone.

7. Procédé suivant la revendication 3, dans lequel la chromone est la 7-(3-cyclopropylamino-2-hydroxypropoxy)-2,3-diphénylchromone.

8. Procédé suivant la revendication 1 ou 2, dans lequel la chromone est le chlorhydrate de 7-(2-hydroxy-3-isopropylaminopropoxy)-3-phénylchromone.

9. Procédé suivant la revendication 1 ou 2, dans lequel la chromone est le chlorhydrate de 7-(2-hydroxy-3-isopropylaminopropoxy)-2-méthylisoflavone.

10. Procédé suivant la revendication 1 ou 2, dans lequel la chromone est le méthiodure de 7-(3-N,N-diméthyl-N-isopropylamino-2-hydroxypropoxy)-2-méthylisoflavone.

11. Procédé suivant la revendication 1 ou 2, dans lequel la chromone est le chlorhydrate de 7-(2-hydroxy-3-isopropylaminopropoxy)-2-trifluorométhylisoflavone.

12. Procédé suivant la revendication 2, dans lequel $R_1$ est un groupe n-propyle et $R_5$ est l'hydrogène.

13. Procédé suivant la revendication 3, dans lequel le sel formé est le chlorhydrate.

14. Procédé suivant la revendication 3, dans lequel le sel formé est le maléate.

15. Procédé de production de la chromone de formule 2, de formule (I)

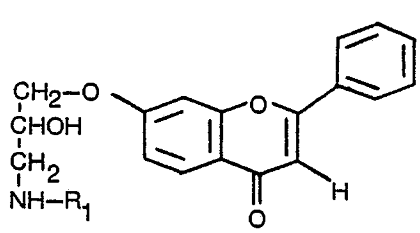

ou d'un sel pharmaceutiquement acceptable de ce composé, formule dans laquelle $R_1$ est un groupe n-propyle ou cyclopropyle, caractérisé par la réaction de l'épichlorhydrine ou de l'épibromhydrine avec un composé de formule

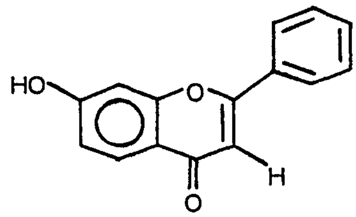

en présence d'un solvant et d'une base pour former un époxyde de la formule ci-dessus, dans laquelle le groupe hydroxyle est remplacé par

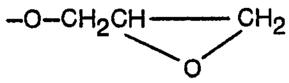

et la réaction de cet époxyde avec une amine de formule $R_1NH_2$ dans laquelle $R_1$ est tel que défini ci-dessus, dans un solvant alcoolique à température élevéee pour obtenir un produit de formule (I), et la transformation facultative de l'amine en son sel pharmaceutiquement acceptable.

16. Procédé suivant la revendication 15, dans lequel est préparé le chlorhydrate de 7-(3-cyclopropylamino-2-hydroxypropoxy)flavone.

17. Procédé suivant la revendication 15, dans lequel est préparé le chlorhydrate de 7-(3-n-propylamino-2-hydroxypropoxy)flavone.

18. Procédé suivant la revendication 15, dans lequel sont préparés les maléates des chromones.